# EUROPEAN PATENT SPECIFICATION

(11) **EP 3 952 987 B1**
(45) Date of publication and mention of the grant of the patent: **18.02.2026**
(21) Application number: 20787593.1
(22) Date of filing: 07.04.2020
(51) Int. Cl.: A61N 5/10, A61K 49/04, A61B 6/00, A61K 41/00, A61K 49/00

(54) **SYSTEM FOR OPTIMIZING RADIOTHERAPHY TREATMENTS**
SYSTEM ZUR OPTIMIERUNG VON STRAHLENTHERAPEUTISCHEN BEHANDLUNGEN
SYSTÈME D'OPTIMISATION DE RADIOTHÉRAPIES

(30) Priority: 08.04.2019 IL 26591419
(43) Date of publication of application: 16.02.2022
(73) Proprietor: Convergent R.N.R Ltd., 3508409 Haifa (IL)
(72) Inventor: ZUCK, Asaf, Vardon (IL); BURSHTEIN, Zeev, 7404611 Nes-Ziona (IL); BAR-DAVID, Aharon, 3680339 Nesher (IL); HAREL, Zeev, 4435846 Kfar Saba (IL); KLECKNER, Michael, 3005213 Ramat-Yishai (IL); BORUKHIN, Shirly, 3032803 Atlit (IL)
(74) Representative: ABG Intellectual Property Law, S.L.
(86) International application number: PCT/IL2020/050418
(87) International publication number: WO 2020/208631

(56) References cited:
- EP-A1- 4 030 164
- WO-A1-2013/121418
- DE-A1- 102007 018 102
- US-A- 5 981 180
- US-A1- 2004 092 807
- US-A1- 2010 246 767
- US-A1- 2012 265 050
- US-A1- 2015 182 118
- US-A1- 2016 252 471
- US-A1- 2017 231 903
- DI, ZICHAO WENDY ET AL.: "JOINT RECONSTRUCTION OF X-RAY FLUORESCENCE AND TRANSMISSION TOMOGRAPHY", OPTICS EXPRESS, vol. 25, no. 12, 12 June 2017 (2017-06-12), pages 13107 - 13124, XP055748410
- DE LA VEGA J.C. ET AL.: "Contrast Media & Molecular Imaging", UTILIZATION OF NANOPARTICLES AS X?RAY CONTRAST AGENTS FOR DIAGNOSTIC IMAGING APPLICATIONS, vol. 10, no. 2, 30 April 2015 (2015-04-30), pages 81 - 95, XP055748419
- MAGGIORELLA L. ET AL., NANOSCALE RADIOTHERAPY WITH HAFNIUM OXIDE NANOPARTICLES. FUTURE ONCOL, vol. 8, no. 9, 31 December 2012 (2012-12-31), pages 1167 - 81, XP002698557
- "Collimation versus Conversion", MERCYBEAM WEBSITE, 20 March 2019 (2019-03-20), XP055748423, Retrieved from the Internet <URL:https://web.archive.org/web/20190320050910/https://www.mercybeam.com/mercy-beam-solutions/collimation-versus-conversion> [retrieved on 20200730]
- CONG, WENXIANG ET AL.: "X-ray micromodulated luminescence tomography in dual-cone geometry", JOURNAL OF BIOMEDICAL OPTICS, vol. 19, no. 7, 31 December 2014 (2014-12-31), pages 76002, XP060047490

## Description

### FIELD OF THE INVENTION

The present invention relates to systems of radiotherapy. More specifically, the present invention relates to a system for enhancing and optimizing the effects of radiotherapy treatments.

### BACKGROUND OF THE INVENTION

Radiation treatments for tumors, especially cancerous tumors, use high-energy particles or waves, such as x-rays, gamma rays, electron beams, or protons, to destroy or damage cancer cells. Such treatments can have long term negative impacts on a patient due to cell damage in non-tumor regions arising from the high levels of radiation used, thus limiting irradiation to lower doses that are not therapeutic.

Various delivery systems and methods of radiation are available nowadays to reduce damage to healthy tissue surrounding the target cells while keeping the irradiation at high therapeutic doses. However, there is still a need for improved system for enhanced radiotherapy treatments that lessen adverse treatment impacts on the patient.

Thus, an aim of the present invention is to provide a system for improving and optimizing the effects of radiotherapy treatments while minimizing damage to healthy tissue surrounding the target organ.

Documents DE 10 2007 018 102 and EP 4 030 164 disclose devices according to the preamble of claim 1.

### SUMMARY

The invention pertains to a radiotherapy treatment system comprising:
(i) an x-ray beam source configurable to deliver an X-ray beam to a target organ,
(ii) optical means for converging and shaping said beam to a cone-shaped X-ray beam of photons which hit the target organ simultaneously,
(iii) multiple high-Z nanoparticles/at least one high-Z fiducial marker attachable to said target organ, said high-Z nanoparticles/at least one high-Z fiducial marker absorbing said X-ray radiation and emitting X-ray fluorescence (XRF) photons,
(iv) at least one XRF detector for detecting said XRF photons ejecting out of a patient's body, and
(v) control means for controlling at least one of components (i)-(iv) for controlling said radiotherapy treatment procedure,
   wherein said x-ray beam focusable on a section in said target organ where the concentration of said high-Z nanoparticles leading to a desirable emission of said XRF photons, and
   wherein in case the emission of said XRF photons decreasing, said x-ray beam is movable to refocus on said section in said target organ where the emission of said XRF photons is desirable.

The optical means comprise at least one converging lens for converging said X-ray beam to said target organ. The invention is defined in the claims. Other embodiments, examples, modifications, methods etc. are not a part of the invention.

Furthermore, in accordance with some embodiments of the present invention, the at least one XRF detector is movable.

Furthermore, in accordance with some embodiments of the present invention, the radiotherapy treatment system further comprising at least one converging lens for converging said XRF photons ejecting out of the patient's body to said at least one XRF detector.

Furthermore, in accordance with some embodiments of the present invention, the at least one XRF detector is selected from a point-sized detector, a one dimensional array detector, and a two-dimensional array detector.

Furthermore, in accordance with some embodiments of the present invention, the point detector is selected from ion chamber type detectors, scintillation detectors and semi-conductor detectors.

Furthermore, in accordance with some embodiments of the present invention, the two-dimensional array detector is a gamma camera.

Furthermore, in accordance with some embodiments of the present invention, the high-Z nanoparticles are selected from metal elements with an atomic number of at least 22.

Furthermore, in accordance with some embodiments of the present invention, the high-Z nanoparticles are selected from titanium (Z=22), vanadium (Z=23), chromium (Z=24), manganese (Z=25), Iron (Z=26), cobalt (Z=27), Nickel (Z=28), copper (Z=29), zinc (Z=30), gallium (Z=31), germanium (Z=32), arsenic (Z=33), selenium (Z=34), bromine (Z=35), rubidium (Z=37), strontium (Z=38), yttrium (Z=39), zirconium (Z=40), niobium (Z=41), molybdenum (Z=42), technetium (Z=43), ruthenium (Z=44), rhodium (Z=45), palladium (Z=46), silver (Z=47), cadmium (Z=48), indium (Z=49), tin (Z=50), antimony (Z=51), tellurium (Z=52), iodine (Z=53), cesium (Z=55), barium (Z=56), lanthanum (Z=57), cerium (Z=58), praseodymium (Z=59), neodymium (Z=60), promethium (Z=61), samarium (Z=62), europium (Z=63), gadolinium (Z=64), terbium (z=65), dysprosium (Z=66) holmium (Z=67), erbium (Z=68), thulium (Z=69) ytterbium (Z=70), lutetium (Z=71), hafnium (Z=72), tantalum (Z=73), tungsten (Z=74), rhenium (Z=75), osmium (Z=76), iridium (Z=77), platinum (Z=78), gold (Z=79),thallium (Z=81), lead (Z=82), bismuth (Z=83), uranium (Z=92).

Furthermore, in accordance with some embodiments of the present invention, the high-Z nanoparticles are preferably selected from Thulium (Z=69) and Erbium (Z=68).

Furthermore, in accordance with some embodiments of the present invention, the high-Z nanoparticles comprise at least one non-metal element.

Furthermore, in accordance with some embodiments of the present invention, the at least one non-metal element is selected from silicone, carbon, halogens, oxygen, and hydrogen.

Furthermore, in accordance with some embodiments of the present invention, the high-Z nanoparticles have a form of nanoscale metal-organic frameworks (nMOFs). Furthermore, in accordance with some embodiments of the present invention, the at least one high-Z nanoparticles comprise Hafnium oxide HfO₂.

Furthermore, in accordance with some embodiments of the present invention, at least two different high-Z nanoparticles are usable, high-Z nanoparticles A and high-Z nanoparticles B, said high-Z nanoparticles A attachable to molecules having affinity to cells of a first type, said high-Z nanoparticles B attachable to molecules having affinity to cells of a second type, wherein the XRF radiation producable by said high-Z nanoparticles A is distinguishable from the XRF radiation producable by said high-Z nanoparticles B.

Furthermore, in accordance with some embodiments of the present invention, in case the XRF radiation producable by said high-Z nanoparticles B decreases and/or the XRF radiation producable by said high-Z nanoparticles A increases during said radiographic treatment procedure, said X-ray beam has to be refocused.

Furthermore, in accordance with some embodiments of the present invention, the cells of a first type being healthy cells and said cells of a second type being non-healthy cells.

Furthermore, in accordance with some embodiments of the present invention, the at least one X-ray detector monitoring in real-time said radiotherapy treatment, thus, providing the distribution of said high-Z nanoparticles in said target organ continuously throughout the radiotherapy treatment.

Furthermore, in accordance with some embodiments of the present invention, the radiotherapy treatment system further comprising a simulation system for simulating said radiographic treatment procedure to maximize the accuracy of the treatment, said simulation system operates independently of the radiotherapy treatment system.

Furthermore, in accordance with some embodiments of the present invention, the simulation system comprising an x-ray source, at least one x-ray detector, and multiple high-Z nanoparticles attachable to said target organ.

Furthermore, in accordance with some embodiments of the present invention, the radiotherapy system producing 3D diagnostic images of said target organ, thus, enabling precise treatments.

Furthermore, in accordance with some embodiments of the present invention, the simulation system and said radiotherapy treatment system are usable interchangeably during a treatment to maximize the accuracy of the treatment.

Furthermore, in accordance with some embodiments of the present invention, the treatment system comprises an x-ray detector for detecting said x-ray beam passing through said target organ for simulating said radiographic treatment.

### BRIEF DESCRIPTION OF THE FIGURES

Fig. 1 illustrates a radiotherapy treatment system for conducting a radiographic X-ray imaging on a target organ during a radiation treatment in accordance with some embodiments of the present invention.
Fig. 2 illustrates a hybrid system having both simulation and treatment capabilities in accordance with some embodiments of the present invention.
Fig. 3 illustrates a radiotherapy simulation system for simulating radiographic treatments to enable precise radiotherapy treatments in accordance with some embodiments of the present invention.
Fig. 4 illustrates a flowchart of a non-claimed method for conducting XRF imaging on a target organ during a radiation treatment.

### DETAILED DESCRIPTION OF THE FIGURES

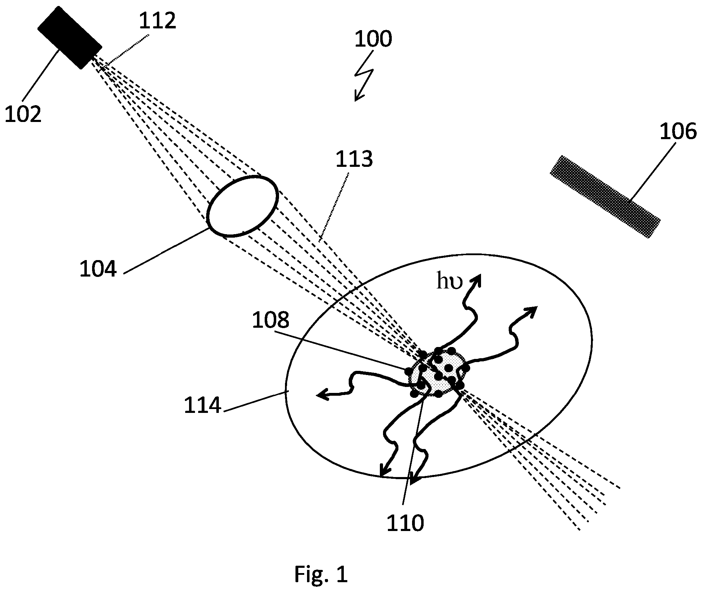

Fig. 1 illustrates a radiotherapy treatment system 100 for conducting a radiographic X-ray imaging on a target organ during a radiation treatment in accordance with some embodiments of the present invention.

Radiotherapy treatment system 100 is used for treating target organs which can be a cancerous tumor or a non-cancerous lesion or other organ while at the same time monitoring in real-time the radiotherapy process and verifying that the radiation "snap" is directed towards a desired location.

Radiotherapy treatment system 100 of the present invention comprises an X-ray beam source 102, a converging lens 104, at least one X-ray detector 106, and either multiple high-Z nanoparticles 108 or at least one high-Z fiducial marker 109.

Radiotherapy treatment system 100 may also include an additional converging lens for converging the emitted XRF photons towards X-ray detector 106.

In accordance with some embodiments of the present invention, high-Z nanoparticles 108 or at least one high-Z fiducial marker 109 are inside and/or on the outside surface of target organ 110.

In accordance with some embodiments of the present invention, all components of radiotherapy treatment system 100 are spatially movable to various target organs in the body (e.g., from head to toe). In accordance with some embodiments of the present invention, at least one X-ray detector 106 may be spatially movable in order to monitor the radiation therapy from various positions around the patient. Alternatively, multiple units of the at least one X-ray detector 106 may be stationary at various positions in the vicinity of the patient for monitoring the radiation therapy from various positions around the patient.

The at least one X-ray detector 106 may be selected from a point-sized detector such as an ion chamber type detector, a scintillating detector, or a semi-conductor detector. Alternatively, the at least one X-ray detector 106 may be selected from a line array detector, and a two-dimensional array detector such as a gamma camera.

Unlike point detectors which have to be moved in space to get mapping, line array detectors and two-dimensional detectors give instantaneous spatial data of the XRF emitted from the body. Such data is highly essential for directing and monitoring the radiation therapy.

In accordance with some embodiments of the present invention, the X-ray beam source 102 emits a diverging X-ray beam 112 which is targeted towards a first converging lens 104 that converges the beam towards a target organ 110 within a patient's body 114.

Prior to the radiation treatment, target organ 110 is administrated with either at least one high-Z fiducial marker 109 or high-Z nanoparticles 108 selected from metal elements with an atomic number of at least 22, including but not limited to titanium (Z=22), vanadium (Z=23), chromium (Z=24), manganese (Z=25), Iron (Z=26), cobalt (Z=27), Nickel (Z=28), copper (Z=29), zinc (Z=30), gallium (Z=31), germanium (Z=32), arsenic (Z=33), selenium (Z=34), bromine (Z=35), rubidium (Z=37), strontium (Z=38), yttrium (Z=39), zirconium (Z=40), niobium (Z=41), molybdenum (Z=42), technetium (Z=43), ruthenium (Z=44), rhodium (Z=45), palladium (Z=46), silver (Z=47), cadmium (Z=48), indium (Z=49), tin (Z=50), antimony (Z=51), tellurium (Z=52), iodine (Z=53), cesium (Z=55), barium (Z=56), lanthanum (Z=57), cerium (Z=58), praseodymium (Z=59), neodymium (Z=60), promethium (Z=61), samarium (Z=62), europium (Z=63), gadolinium (Z=64), terbium (z=65), dysprosium (Z=66) holmium (Z=67), erbium (Z=68), thulium (Z=69) ytterbium (Z=70), lutetium (Z=71), hafnium (Z=72), tantalum (Z=73), tungsten (Z=74), rhenium (Z=75), osmium (Z=76), iridium (Z=77), platinum (Z=78), gold (Z=79), thallium (Z=81), lead (Z=82), bismuth (Z=83), uranium (Z=92).

In accordance with some embodiments of the present invention, the use of high-Z nanoparticles of either non-toxic Thulium (Z=69) or Erbium (Z=68) which has low toxicity is preferred in some cases due to a good match between the K-edge absorption resonance energy of these materials, with the emission characteristic of Tungsten. The K-edge of Tellurium - Tm is 59.3896 keV, the K-edge of Erbium - Er is 57.4855 keV, and the Ka emission of Tungsten, which is the most commonly used anode material in X-ray tubes for medical applications, is 59.3 keV.

It should be noted that non-metal components such as silicone, carbon. halogens (such as iodine), oxygen, and even hydrogen may be present in the compound, an example is the Hafnium oxide HfO₂.

It should be further noted that the high-Z nanoparticles may appear in the form of nanoscale metal-organic frameworks (nMOFs).

In accordance with some embodiments of the present invention, the distribution of such nanoparticles is obtained during the radiation treatment via the at least one X-ray detector 106 detecting the emitted XRF photons from said high-Z nanoparticles 108. The high-Z nanoparticles 108/high-Z fiducial marker 109 absorb the X-ray radiation and emit X-ray fluorescence (XRF) photons where a fraction of sufficiently high energy photons emerges out of the patient's body and detected by X-ray detector 106.

The XRF emission intensity depends on the energy and intensity of the radiation and the concentration of the high-Z nanoparticles 108. In the simplest case, it is assumed that the XRF emission is maximal when the converged radiation focal point hits the highest concentration of high-Z nanoparticles 108.

In accordance with some embodiments of the present invention, the at least one X-ray detector 106 provides information about the actual distribution of nanoparticles, i.e., monitors a real-time radiotherapy process to provide the distribution of nanoparticles in the area of interest continuously throughout the radiotherapy treatment. Such information is highly essential for verifying that the radiation "snap" is indeed directed towards the exact location (spatial coordination), i.e., towards the target organ having a high density of high-Z nanoparticles 108.

When the XRF emission decreases, it is required to check whether the beam's focal point is in the correct place. If the beam is out of focus, it has to be directed to the section where the XRF radiation received is at a desirable value.

In accordance with some embodiments of the present invention, the use of a converging X-ray beam 113 and high-Z nanoparticles 108 enables the target organ 110 to receive a large dosage while the distal and preceding organs receive minimal dosage. Minimizing the dosage to healthy tissues reduces the amount of side effects that the patient will suffer from. Therefore, the use of a converging beam 113 and high-Z nanoparticles 108 reduces long term as well as short term side effects. In addition, the increased dose supplied to the target organ 110 increases the efficiency of the therapy and might even shorten the therapy course making it more tolerable to patients.

In accordance with some embodiments of the present invention, target organ 110 may be administrated with high-Z nanoparticles 108 of various elements. Prior to being administrated, high-Z nanoparticles 108A of certain element(s) may be attached to molecules having an affinity to cells of a first type while high-Z nanoparticles 108B of other element(s) may be attached to molecules having an affinity to cells of a second type. Since high-Z nanoparticles 108 of different elements produce different XRF radiations (different energies), such configuration enables determining the particle distribution in cells of both types and focusing the X-ray beam 112 on the desired cells.

For instance, high-Z nanoparticles 108A of certain element(s) may be attached to molecules having an affinity to healthy cells while high-Z nanoparticles 108B of other element(s) may be attached to molecules having an affinity to non-healthy cells. As long as the beam is focused, the XRF emitted from the high-Z nanoparticles 108B may be dominant. However, if during the treatment procedure the XRF emitted from the high-Z nanoparticles 108B is no longer dominant, i.e., if the XRF emitted from high-Z nanoparticles 108B decreases and/or the XRF emitted from high-Z nanoparticles 108A increases, it is most likely due to a beam shift from the initial location, and thus, the beam has to be refocused to its original location. In accordance with some embodiments of the present invention, radiotherapy treatment system 100 may include a simulator for providing a radiographic reflection of the distribution of high-Z nanoparticles 108 in target organ 110, thus, for verifying treatment plans with high accuracy.

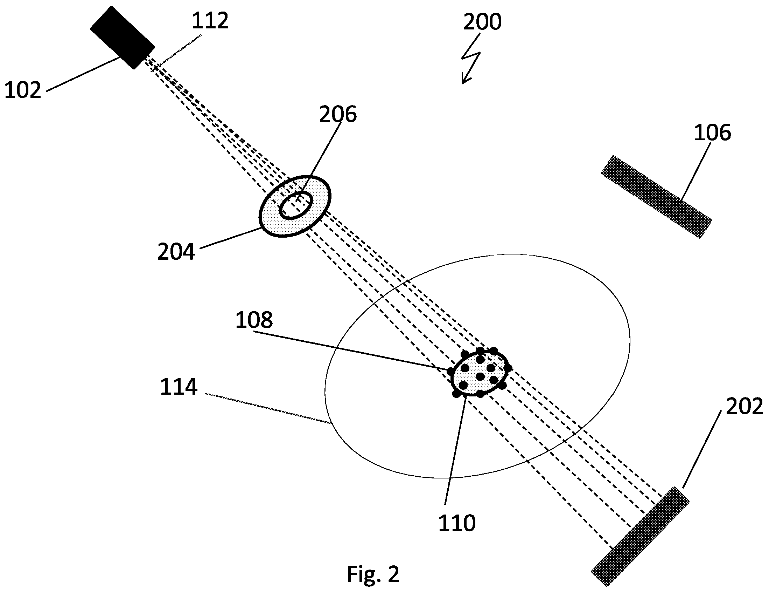

Fig. 2 illustrates a hybrid system 200 having both simulation and treatment capabilities in accordance with some embodiments of the present invention. Hybrid system 200 is basically the radiotherapy treatment system 100 of Fig. 1 modified to implement simulation capabilities for simulating of target organ 110, and thus, for enabling precise treatment procedures.

Hybrid system 200 can quickly switch between a radiographic simulation mode and a radiotherapy X-ray treatment mode without moving the patient from the actual treatment position, i.e., patients can be imaged in the true treatment position.

Hybrid system 200 comprises a converging X-ray beam source 102, at least one X-ray detector 106, target organ 110 to be detected by X-ray detector 202, high-Z nanoparticles 108 or at least one high-Z fiducial marker 109 attached inside and/or on the outside surface of the target organ 110, lens 204, and a second detector 202 for detecting beam 112 traveling through lens 204during simulations runs.

In accordance with some embodiments of the present invention, lens 204 comprises an aperture 206 through which X-ray Beam 112 passes towards target organ 110 and detected via X-ray detector 202 during simulation runs.

In accordance with some embodiments of the present invention, simulations are run prior to a radiation treatment for focusing the X-ray beam 112 without moving the patient from the actual treatment position.

In order to avoid damages to target organ 110 or any other organs in the vicinity of the target organ 110, X-ray beam 112 passes through aperture 206 of lens 204, and thus, does not converge into the organ during the simulation process.

In accordance with some embodiments of the present invention, hybrid system 200 produces 3D diagnostic images of target organ 110, and thus, enables precise treatments. At the end of the simulation process, System 200 is being changed from radiographic simulating mode for beam focusing to a radiotherapy X-ray treatment mode without moving the patient from the actual treatment position.

In accordance with some embodiments of the present invention, hybrid system 200 can quickly change from a radiographic simulation mode to a radiotherapy X-ray treatment mode and vice versa without moving the patient from the actual treatment position, i.e., patients can be imaged in the true treatment position.

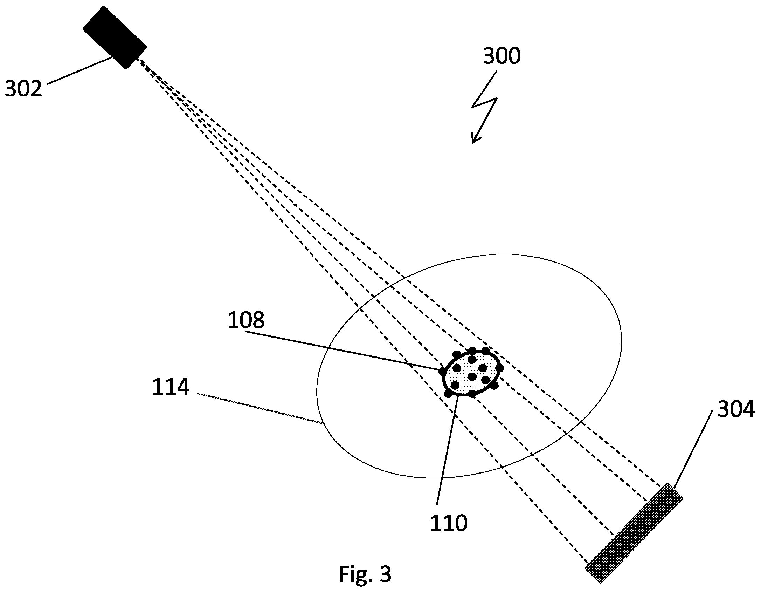

Fig. 3 illustrates a radiotherapy simulation system 300 for simulating radiographic treatments in accordance with some embodiments of the present invention.

In this case, radiotherapy simulation system 300 is a separate, stand-alone system which operates independently of radiotherapy treatment system 100.

Radiotherapy simulation system 300 comprising an X-ray beam source 302 which is not that of the therapeutic beam but rather an independent beam source 302, at least one X-ray detector 304, and high-Z nanoparticles 108/at least one high-Z fiducial marker 109 attached inside and on the outside surface of the target organ 110.

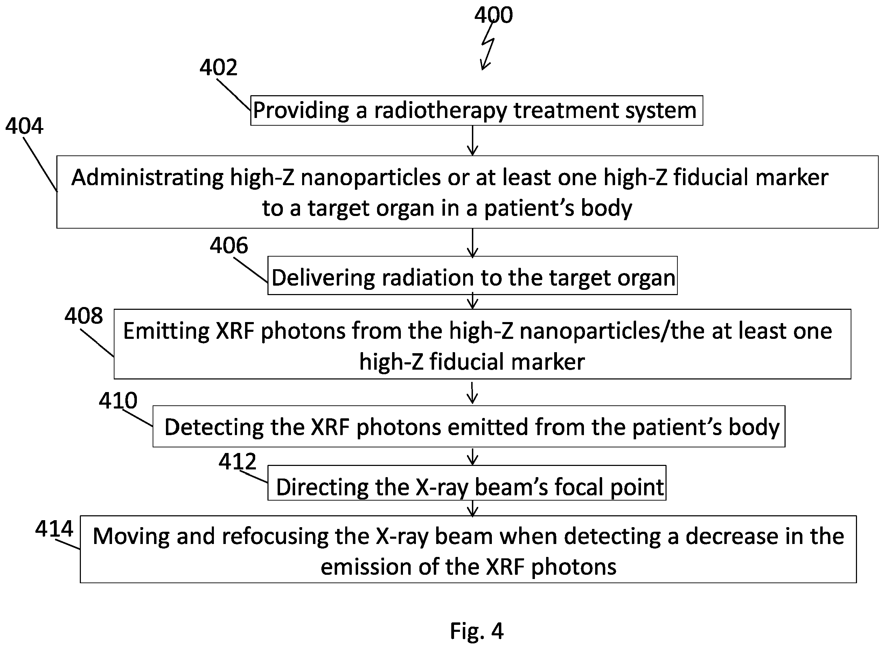

In accordance with some embodiments of the present invention, radiotherapy treatment system 100 of Fig. 1 and radiotherapy simulation system 300 may be used interchangeably during a treatment in order to maximize the accuracy of the treatment. Fig. 4 illustrates a flowchart of a method 400 for conducting XRF imaging on a target organ 110 during a radiation treatment.

The method 400 begins with step 402 in which a radiotherapy treatment system is provided.

In administering step 404, an effective amount of high-Z nano-particles 108 or at least one high-Z fiducial marker 109 is localized in the target organ 110 via (a) injection into the blood stream with or without affinity agents to aid in localization, (b) direct injection into the target organ 110 or any other administering step that is capable of localizing an effective amount of the high-Z nanoparticles 108 in the target organ 110. X-ray radiation is delivered to the target organ 110 in step 406. As radiation is applied to the target organ 110 and absorbed by the high-Z nano-particles 108, a portion of the radiation produces energized electrons such as photoelectrons, Compton electrons, or the like, is ejected from the NP and deliver their energy to the target organ.

Another process is X ray fluorescence (XRF) where photons having specific energies are emitted from the high-Z nano-particles 108 or the at least one high-Z fiducial marker 109 in step 408. The emitted energy is delivered via energetic electrons and photons to the surrounding environment (the tumor), thereby increasing the efficiency of therapeutic dose delivering to the tumor - the photons and electrons are absorbed by the tissues of the target organ resulting in cell death or a reduction or elimination of a tumor in a reducing cancer cells.

A fraction of sufficiently high energy XRF photons emerges out of the patient's body 114 and collected by at least one detector 106 located at different positions in the patient vicinity in step 410 to determine the exact distribution of the nanoparticles 108.

The focal point of the X-ray beam 112 is directed to the target area in step 412 as follows:
- X-ray radiation is delivered to the target organ 110,
- the primary (therapeutic) X ray beam 112 causes an emission of XRF photons via interaction of the high-Z nanoparticles 108,
- photons ejected from the patient's body 114 are detected continuously throughout the treatment, the X-ray beam 112 is focused on the target organ 110 and preferably on a section in the target organ 110 where the concentration of the high-Z nanoparticles 108 lead to a desirable emission of the XRF photons.

Thus, in accordance with some examples, the XRF photons ejected from the human's body are detected continuously throughout the radiotherapy treatment, and the X-ray beam is automatically refocused whenever a decrease in the emission of the XRF photons is detected.

When the reading of detector 106 is less than a pre-defined value, it is assumed that X-ray beam 112 either hits a region within the target organ 110 where the concentration of high-Z nanoparticles 108 is undesirable or there is greater absorption in the patient's body 114. This means that the focus position is not optimal and therefore the direction of X-ray beam 112 is corrected in step 414.

Returning the X-ray beam 112 to its original location involves with scanning the target organ 110 to locate the point where the XRF emission is desirable and returning the therapeutic radiation to the new coordinates.

Method 400 may further comprise simulation runs to obtain a distribution of the high-Z nanoparticles 108 in the target organ 110 prior to delivering therapeutic radiation to the target organ. Alternatively, the skin may be marked via markers or tattoos to help directing the beam112 to a desired location.

The desired focal point of the beam 112, is determined via straight-forward triangulation in step 410. Triangulation is a process of determining the location of a point by forming triangles to it from known points. Basically, the configuration consists of either a single movable sensor or multiple sensors for observing the item, i.e., the XRF emitted from said high-Z nanoparticles. One of the sensors is typically a point detector, or one-dimensional array detector, or two-dimensional array detector, and the other sensor may be either a point detector or one-dimensional array detector, or two-dimensional array detector or a new location of the detector and the third is the XRF emitter. The projection centers of the sensors and a considered point on the NP loaded target organ's surface define a triangle. Within this triangle, the distance between the sensors is the base b and must be known. By determining the angles between the projection rays of the sensors and the basis, the intersection point, and thus the 3D coordinate, is calculated from the triangular relations.

## Claims

1. A radiotherapy treatment system (100) for conducting radiographic X-ray imaging on a target organ during radiographic treatment to the target organ comprising:
(i) an x-ray beam source (102) configurable to deliver an X-ray beam (112) to a target organ (110),
(ii) optical means for converging and shaping said beam to a cone-shaped X-ray beam (112) of photons which hit the target organ (110) simultaneously,
(iii) multiple high-Z nanoparticles (108) administrable to the target organ or at least one high-Z fiducial marker attachable to said target organ (110), said high-Z nanoparticles (108) or at least one high-Z fiducial marker absorbing said X-ray radiation and emitting X-ray fluorescence (XRF) photons,
(iv) at least one XRF detector (106) for detecting said XRF photons ejecting out of a patient's body, and
(v) control means for controlling at least one of components (i)-(iv) for controlling said radiotherapy treatment procedure,
wherein said X-ray beam (112) is focusable on a section in said target organ (110) having the concentration of said high-Z nanoparticles (108) leading to a desirable emission of said XRF photons, and
wherein in case the emission of said XRF photons decreasing, said X-ray beam (112) is movable to refocus on said section in said target organ (110) where the emission of said XRF photons is desirable;
**characterised in that**
the optical means comprise at least one converging lens (104, 204) for converging said X-ray beam (112) to said target organ (110).

2. The radiotherapy treatment system (100) of Claim 1, wherein said at least one XRF detector (106) is movable.

3. The radiotherapy treatment system (100) of Claim 1, further comprising at least one converging lens (104) for converging said XRF photons ejecting out of the patient's body to said at least one XRF detector (106).

4. The radiotherapy treatment system (100) of any one of Claims 1-3, wherein said at least one XRF detector (106) is selected from a point-sized detector, a one-dimensional array detector, and a two-dimensional array detector.

5. The radiotherapy treatment system (100) of Claim 4, wherein said point detector is selected from ion chamber type detectors, scintillation detectors and semi-conductor detectors.

6. The radiotherapy treatment system (100) of Claim 5, wherein said two-dimensional array detector is a gamma camera.

7. The radiotherapy treatment system (100) of Claim 1, wherein said high-Z nanoparticles (108) are selected from metal elements with an atomic number of at least 22.

8. The radiotherapy treatment system (100) of Claim 7, wherein said high-Z nanoparticles are selected from titanium (Z=22), vanadium (Z=23), chromium (Z=24), manganese (Z=25), Iron (Z=26), cobalt (Z=27), Nickel (Z=28), copper (Z=29), zinc (Z=30), gallium (Z=31), germanium (Z=32), arsenic (Z=33), selenium (Z=34), bromine (Z=35), rubidium (Z=37), strontium (Z=38), yttrium (Z=39), zirconium (Z=40), niobium (Z=41), molybdenum (Z=42), technetium (Z=43), ruthenium (Z=44), rhodium (Z=45), palladium (Z=46), silver (Z=47), cadmium (Z=48), indium (Z=49), tin (Z=50), antimony (Z=51), tellurium (Z=52), iodine (Z=53), cesium (Z=55), barium (Z=56), lanthanum (Z=57), cerium (Z=58), praseodymium (Z=59), neodymium (Z=60), promethium (Z=61), samarium (Z=62), europium (Z=63), gadolinium (Z=64), terbium (z=65), dysprosium (Z=66) holmium (Z=67), erbium (Z=68), thulium (Z=69) ytterbium (Z=70), lutetium (Z=71), hafnium (Z=72), tantalum (Z=73), tungsten (Z=74), rhenium (Z=75), osmium (Z=76), iridium (Z=77), platinum (Z=78), gold (Z=79),thallium (Z=81), lead (Z=82), bismuth (Z=83), uranium (Z=92).

9. The radiotherapy treatment system (100) of Claim 8, wherein said high-Z nanoparticles (108) are selected from Thulium (Z=69) and Erbium (Z=68).

10. The radiotherapy treatment system (100) of any one of Claims 1, 7-9, wherein said high-Z nanoparticles (108) comprise at least one non-metal element.

11. The radiotherapy treatment system (100) of Claim 10, wherein said at least one non-metal element is selected from silicone, carbon, halogens, oxygen, and hydrogen.

12. The radiotherapy treatment system (100) of any one of Claims 1, and 7-11, wherein said high-Z nanoparticles (108) have a form of nanoscale metal-organic frameworks, nMOFs.

13. The radiotherapy treatment system (100) of any one of Claims 1, 7-9, wherein said at least one high-Z nanoparticles (108) comprise Hafnium oxide HfO₂.

14. The radiotherapy treatment system (100) of any one of Claims 1, 7-13, comprising at least two different high-Z nanoparticles (108): high-Z nanoparticles A and high-Z nanoparticles B, said high-Z nanoparticles A being attachable to molecules having affinity to cells of a first type, said high-Z nanoparticles B being attachable to molecules having affinity to cells of a second type, wherein the XRF radiation producible by said high-Z nanoparticles A is distinguishable from the XRF radiation producible by said high-Z nanoparticles B.

15. The radiotherapy treatment system (100) of Claim 14, wherein in case the XRF radiation producible by said high-Z nanoparticles B decreases and/or the XRF radiation producible by said high-Z nanoparticles A increases during said radiographic treatment procedure, said X-ray beam (112) has to be refocused.

16. The radiotherapy treatment system (100) of any one of Claims 14-15, said cells of a first type being healthy cells and said cells of a second type being non-healthy cells.

17. The radiotherapy treatment system (100) of any one of Claims 1, 2-6, wherein said at least one X-ray detector (106) is adapted to monitor in real-time said radiotherapy treatment, thus, providing the distribution of said high-Z nanoparticles (108) in said target organ (110) continuously throughout the radiotherapy treatment.

18. The radiotherapy treatment system (100) of any one of Claims 1-17, further comprising a simulation system (300) for simulating said radiographic treatment procedure to maximize the accuracy of the treatment, said simulation system operating independently of the radiotherapy treatment system.

19. The radiotherapy treatment system (100) of Claim 18, wherein said simulation system (300) comprises an x-ray source (302), at least one x-ray detector (304), and multiple high-Z nanoparticles (108) attachable to said target organ (110).

20. The radiotherapy treatment system (100) of any one of Claims 17-19, wherein said radiotherapy system is adapted to produce 3D diagnostic images of said target organ (110), thus, enabling precise treatments.

21. The radiotherapy treatment system (100) of any one of Claims 18-20, wherein said simulation system (300) and said radiotherapy treatment system (100) are usable interchangeably during a treatment to maximize the accuracy of the treatment.

22. The radiotherapy treatment system (100, 200) according to claim 1, said radiotherapy treatment system further comprising:
(e) an x-ray detector for detecting said x-ray beam passing through said target organ for simulating said radiographic treatment,
wherein said radiotherapy system (200) is able to switch between a simulation mode and a radiotherapy treatment mode without moving a patient from one position to another.

23. The radiotherapy treatment system (100, 200) of Claim 22, wherein comprise said at least one lens (204) comprises an openable aperture (206), said openable aperture (206) maintained closed for converging said X-ray beam (112) to said target organ (110) during said radiotherapy treatment procedure, said aperture (206) maintained open to allow said X-ray beam (112) to pass through said aperture (206) for simulating said radiographic treatment.

24. The radiotherapy treatment system (100, 200) of Claim 22, wherein said radiotherapy system is adapted to produce 3D diagnostic images of said target organ (110), thus, enabling precise treatments.

25. The radiotherapy treatment system (100, 200) of Claim 22, wherein said at least one XRF detector (202) is movable.

26. The radiotherapy treatment system (100, 200) of Claim 22, further comprising at least one converging lens (204) for converging said XRF photons ejecting out of the patient's body to said at least one XRF detector (202).

27. The radiotherapy treatment system (100, 200) of any one of Claims 22-25, wherein said at least one XRF detector (202) is selected from a point-sized detector, a one dimensional array detector, and a two-dimensional array detector.

28. The radiotherapy treatment system (100, 200) of Claim 26, wherein said point-sized detector is selected from ion chamber type detectors, scintillation detectors and semi-conductor detectors.

29. The radiotherapy treatment system (100, 200) of Claim 26, wherein said two-dimensional array detector is a gamma camera.

30. The radiotherapy treatment system (100, 200) of Claim 22, wherein said high-Z nanoparticles (108) are selected from metal elements with an atomic number of at least 22.

31. The radiotherapy treatment system (100, 200) of Claim 30, wherein said high-Z nanoparticles (108) are selected from titanium (Z=22), vanadium (Z=23), chromium (Z=24), manganese (Z=25), Iron (Z=26), cobalt (Z=27), Nickel (Z=28), copper (Z=29), zinc (Z=30), gallium (Z=31), germanium (Z=32), arsenic (Z=33), selenium (Z=34), bromine (Z=35), rubidium (Z=37), strontium (Z=38), yttrium (Z=39), zirconium (Z=40), niobium (Z=41), molybdenum (Z=42), technetium (Z=43), ruthenium (Z=44), rhodium (Z=45), palladium (Z=46), silver (Z=47), cadmium (Z=48), indium (Z=49), tin (Z=50), antimony (Z=51), tellurium (Z=52), iodine (Z=53), cesium (Z=55), barium (Z=56), lanthanum (Z=57), cerium (Z=58), praseodymium (Z=59), neodymium (Z=60), promethium (Z=61), samarium (Z=62), europium (Z=63), gadolinium (Z=64), terbium (z=65), dysprosium (Z=66) holmium (Z=67), erbium (Z=68), thulium (Z=69) ytterbium (Z=70), lutetium (Z=71), hafnium (Z=72), tantalum (Z=73), tungsten (Z=74), rhenium (Z=75), osmium (Z=76), iridium (Z=77), platinum (Z=78), gold (Z=79),thallium (Z=81), lead (Z=82), bismuth (Z=83), uranium (Z=92).

32. The radiotherapy treatment system (100, 200) of Claim 31, wherein said high-Z nanoparticles (108) are selected from Thulium (Z=69) and Erbium (Z=68).

33. The radiotherapy treatment system (100, 200) of any one of Claims 22, 31, & 32, wherein said high-Z nanoparticles (108) comprise at least one non-metal element.

34. The radiotherapy treatment system (100, 200) of Claim 33, wherein said at least one non-metal element is selected from silicone, halogens, oxygen, and hydrogen.

35. The radiotherapy treatment system (100, 200) of any one of Claims 22, 31-32, wherein said at least one high-Z nanoparticles (108) comprise Hafnium oxide HfO₂.

36. The radiotherapy treatment system (100, 200) of any one of Claims 22 and 31-35, comprising at least two types, a first type and a second type, of said high-Z nanoparticles (108), said high-Z nanoparticles (108) of said first type being attached to molecules having affinity to one kind (e.g. healthy cells), said high-Z nanoparticles (108) of said second type being attached to molecules having affinity to other kind (e.g. non-healthy) of cells, so that, the XRF radiation produced by said high-Z nanoparticles of said first type is distinguishable from the XRF radiation produced by said high-Z nanoparticles (108) of said second type.

37. The radiotherapy treatment system (100, 200) of any one of Claims 21, 24-28, wherein said at least one X-ray detector (202) is adapted to monitor in real-time said radiotherapy treatment procedure, thus, providing the distribution of said high-Z nanoparticles (108) in said target organ (110) continuously throughout the radiotherapy treatment.

## Patentansprüche

1. Das Strahlentherapiebehandlungssystem (100) zur Durchführung einer radiografischen Röntgenbildgebung an einem Zielorgan während einer radiografischen Behandlung des Zielorgans, umfassend:
(i) eine Röntgenstrahlquelle (102), die so konfigurierbar ist, dass sie einen Röntgenstrahl (112) an ein Zielorgan (110) liefert,
(ii) optische Mittel zum Bündeln und Formen des Strahls zu einem kegelförmigen Röntgenstrahl (112) aus Photonen, die gleichzeitig auf das Zielorgan (110) treffen,
(iii) mehrere Nanopartikel mit hohem Z-Wert (108), die dem Zielorgan verabreicht werden können, oder mindestens einen Referenzmarker mit hohem Z-Wert, der an dem Zielorgan (110) angebracht werden kann, wobei die Nanopartikel mit hohem Z-Wert (108) oder mindestens ein Referenzmarker mit hohem Z-Wert die Röntgenstrahlung absorbieren und Röntgenfluoreszenzphotonen (XRF) emittieren,
(iv) mindestens einen XRF-Detektor (106) zum Erfassen der aus dem Körper eines Patienten austretenden XRF-Photonen, und
(v) Steuermittel zum Steuern mindestens einer der Komponenten (i) bis (iv) zum Steuern des Strahlentherapiebehandlungsverfahrens,
wobei der Röntgenstrahl (112) auf einen Abschnitt in dem Zielorgan (110) fokussierbar ist, der die Konzentration der Nanopartikel (108) mit hohem Z aufweist, was zu einer gewünschten Emission der XRF-Photonen führt, und
wobei im Falle einer Abnahme der Emission der XRF-Photonen der Röntgenstrahl (112) bewegbar ist, um ihn erneut auf den Abschnitt in dem Zielorgan (110) zu fokussieren, in dem die Emission der XRF-Photonen wünschenswert ist; **dadurch gekennzeichnet, dass** die optischen Mittel mindestens eine Sammellinse (104, 204) zum Sammeln des Röntgenstrahls (112) auf das Zielorgan (110) umfassen.

2. Das Strahlentherapiebehandlungssystem (100) nach Anspruch 1, wobei der mindestens eine XRF-Detektor (106) beweglich ist.

3. Das Strahlentherapiebehandlungssystem (100) nach Anspruch 1, das ferner mindestens eine Sammellinse (104) umfasst, um die aus dem Körper des Patienten austretenden XRF-Photonen auf den mindestens einen XRF-Detektor (106) zu fokussieren.

4. Das Strahlentherapiebehandlungssystem (100) nach einem der Ansprüche 1 bis 3, wobei der mindestens eine XRF-Detektor (106) aus einem punktförmigen Detektor, einem eindimensionalen Array-Detektor und einem zweidimensionalen Array-Detektor ausgewählt ist.

5. Das Strahlentherapiebehandlungssystem (100) nach Anspruch 4, wobei der Punktdetektor aus Ionenkammerdetektoren, Szintillationsdetektoren und Halbleiterdetektoren ausgewählt ist.

6. Das Strahlentherapiebehandlungssystem (100) nach Anspruch 5, wobei der zweidimensionale Array-Detektor eine Gammakamera ist.

7. Das Strahlentherapiebehandlungssystem (100) nach Anspruch 1, wobei die Nanopartikel mit hohem Z-Wert (108) aus Metallelementen mit einer Ordnungszahl von mindestens 22 ausgewählt sind.

8. Das Strahlentherapiebehandlungssystem (100) nach Anspruch 7, wobei die Nanopartikel mit hohem Z-Wert aus Titan (Z = 22), Vanadium (Z = 23), Chrom (Z = 24), Mangan (Z = 25), Eisen (Z = 26), Kobalt (Z = 27), Nickel (Z = 28), Kupfer (Z=29), Zink (Z=30), Gallium (Z=31), Germanium (Z=32), Arsen (Z=33), Selen (Z=34), Brom (Z=35), Rubidium (Z=37), Strontium (Z=38), Yttrium (Z=39), Zirkonium (Z=40), Niob (Z=41), Molybdän (Z=42), Technetium (Z=43), Ruthenium (Z=44), Rhodium (Z=45), Palladium (Z=46), Silber (Z=47), Cadmium (Z=48), Indium (Z=49), Zinn (Z=50), Antimon (Z=51), Tellur (Z=52), Jod (Z=53), Cäsium (Z=55), Barium (Z=56), Lanthan (Z=57), Cer (Z=58), Praseodym (Z=59), Neodym (Z=60), Promethium (Z=61), Samarium (Z=62), Europium (Z=63), Gadolinium (Z=64), Terbium (Z=65), Dysprosium (Z=66), Holmium (Z=67), Erbium (Z=68), Thulium (Z=69), Ytterbium (Z=70), Lutetium (Z=71), Hafnium (Z=72), Tantal (Z=73), Wolfram (Z=74), Rhenium (Z=75), Osmium (Z=76), Iridium (Z=77), Platin (Z=78), Gold (Z=79), Thallium (Z=81), Blei (Z=82), Wismut (Z=83), Uran (Z=92).

9. Das Strahlentherapiebehandlungssystem (100) nach Anspruch 8, wobei die Nanopartikel mit hohem Z-Wert (108) aus Thulium (Z=69) und Erbium (Z=68) ausgewählt sind.

10. Das Strahlentherapiebehandlungssystem (100) nach einem der Ansprüche 1, 7-9, wobei die Nanopartikel mit hohem Z-Wert (108) mindestens ein Nichtmetallelement umfassen.

11. Das Strahlentherapiebehandlungssystem (100) nach Anspruch 10, wobei das mindestens eine Nichtmetallelement aus Silizium, Kohlenstoff, Halogenen, Sauerstoff und Wasserstoff ausgewählt ist.

12. Das Strahlentherapiebehandlungssystem (100) nach einem der Ansprüche 1 und 7-11, wobei die Nanopartikel (108) mit hohem Z-Wert die Form von nanoskaligen metallorganischen Gerüsten (nMOFs) haben.

13. Das Strahlentherapiebehandlungssystem (100) nach einem der Ansprüche 1, 7 bis 9, wobei die mindestens eine Nanopartikel mit hohem Z-Wert (108) Hafniumoxid HfO₂ umfasst.

14. Das Strahlentherapiebehandlungssystem (100) nach einem der Ansprüche 1, 7 bis 13, umfassend mindestens zwei verschiedene Nanopartikel mit hohem Z-Wert (108): Nanopartikel A mit hohem Z-Wert und Nanopartikel B mit hohem Z-Wert, wobei die Nanopartikel A mit hohem Z-Wert an Moleküle gebunden werden können, die eine Affinität zu Zellen eines ersten Typs aufweisen, und die Nanopartikel B mit hohem Z-Wert an Moleküle gebunden werden können, die eine Affinität zu Zellen eines zweiten Typs aufweisen, wobei die von den Nanopartikeln A mit hohem Z-Wert erzeugbare XRF-Strahlung von der von den Nanopartikeln B mit hohem Z-Wert erzeugbaren XRF-Strahlung unterscheidbar ist.

15. Das Strahlentherapiebehandlungssystem (100) nach Anspruch 14, wobei in dem Fall, dass die durch die Nanopartikel B mit hohem Z erzeugbare XRF-Strahlung während des radiologischen Behandlungsverfahrens abnimmt und/oder die durch die Nanopartikel A mit hohem Z erzeugbare XRF-Strahlung zunimmt, der Röntgenstrahl (112) neu fokussiert werden muss.

16. Das Strahlentherapiebehandlungssystem (100) nach einem der Ansprüche 14-15, wobei die Zellen eines ersten Typs gesunde Zellen sind und die Zellen eines zweiten Typs nicht gesunde Zellen sind.

17. Das Strahlentherapiebehandlungssystem (100) nach einem der Ansprüche 1, 2 bis 6, wobei der mindestens eine Röntgendetektor (106) dazu ausgelegt ist, die Strahlentherapie in Echtzeit zu überwachen und so die Verteilung der Nanopartikel (108) mit hohem Z-Wert im Zielorgan (110) während der gesamten Strahlentherapie kontinuierlich bereitzustellen.

18. Das Strahlentherapiebehandlungssystem (100) nach einem der Ansprüche 1 bis 17, das ferner ein Simulationssystem (300) zum Simulieren des radiologischen Behandlungsverfahrens umfasst, um die Genauigkeit der Behandlung zu maximieren, wobei das Simulationssystem unabhängig vom Strahlentherapiebehandlungssystem arbeitet.

19. Das Strahlentherapiebehandlungssystem (100) nach Anspruch 18, wobei das Simulationssystem (300) eine Röntgenquelle (302), mindestens einen Röntgendetektor (304) und mehrere Nanopartikel (108) mit hohem Z-Wert umfasst, die an dem Zielorgan (110) anbringbar sind.

20. Das Strahlentherapiebehandlungssystem (100) nach einem der Ansprüche 17 bis 19, wobei das Strahlentherapiebehandlungssystem dazu ausgelegt ist, 3D-Diagnosebilder des Zielorgans (110) zu erzeugen, wodurch präzise Behandlungen ermöglicht werden.

21. Das Strahlentherapiebehandlungssystem (100) nach einem der Ansprüche 18 bis 20, wobei das Simulationssystem (300) und das Strahlentherapiebehandlungssystem (100) während einer Behandlung austauschbar verwendet werden können, um die Genauigkeit der Behandlung zu maximieren.

22. Das Strahlentherapiebehandlungssystem (100, 200) gemäß Anspruch 1, wobei das Strahlentherapiebehandlungssystem ferner umfasst:
(e) einen Röntgendetektor zum Erfassen des durch das Zielorgan hindurchtretenden Röntgenstrahls zum Simulieren der radiologischen Behandlung,
wobei das Strahlentherapiesystem (200) zwischen einem Simulationsmodus und einem Strahlentherapiemodus umschalten kann, ohne dass der Patient von einer Position in eine andere bewegt werden muss.

23. Das Strahlentherapiebehandlungssystem (100, 200) nach Anspruch 22, wobei die mindestens eine Linse (204) eine öffenbare Blende (206) umfasst, wobei die zu öffnende Blende (206) während des Strahlentherapiebehandlungsverfahrens geschlossen gehalten wird, um den Röntgenstrahl (112) auf das Zielorgan (110) zu fokussieren, und wobei die Blende (206) offen gehalten wird, damit der Röntgenstrahl (112) zur Simulation der radiologischen Behandlung durch die Blende (206) hindurchtreten kann.

24. Das Strahlentherapiebehandlungssystem (100, 200) nach Anspruch 22, wobei das Strahlentherapiesystem dazu ausgelegt ist, 3D-Diagnosebilder des Zielorgans (110) zu erzeugen, wodurch präzise Behandlungen ermöglicht werden.

25. Das Strahlentherapiebehandlungssystem (100, 200) nach Anspruch 22, wobei der mindestens eine XRF-Detektor (202) beweglich ist.

26. Das Strahlentherapiebehandlungssystem (100, 200) nach Anspruch 22, das ferner mindestens eine Sammellinse (204) umfasst, um die aus dem Körper des Patienten austretenden XRF-Photonen auf den mindestens einen XRF-Detektor (202) zu fokussieren.

27. Das Strahlentherapiebehandlungssystem (100, 200) nach einem der Ansprüche 22 bis 25, wobei der mindestens eine XRF-Detektor (202) aus einem punktförmigen Detektor, einem eindimensionalen Array-Detektor und einem zweidimensionalen Array-Detektor ausgewählt ist.

28. Das Strahlentherapiebehandlungssystem (100, 200) nach Anspruch 26, wobei der punktförmige Detektor aus Ionenkammerdetektoren, Szintillationsdetektoren und Halbleiterdetektoren ausgewählt ist.

29. Das Strahlentherapiebehandlungssystem (100, 200) nach Anspruch 26, wobei der zweidimensionale Array-Detektor eine Gammakamera ist.

30. Das Strahlentherapiebehandlungssystem (100, 200) nach Anspruch 22, wobei die Nanopartikel mit hohem Z-Wert (108) aus Metallelementen mit einer Ordnungszahl von mindestens 22 ausgewählt sind.

31. Das Strahlentherapiebehandlungssystem (100, 200) nach Anspruch 30, wobei die Nanopartikel mit hohem Z-Wert (108) aus Titan (Z = 22), Vanadium (Z = 23), Chrom (Z = 24), Mangan (Z = 25), Eisen (Z = 26), Kobalt (Z = 27), Nickel (Z=28), Kupfer (Z=29), Zink (Z=30), Gallium (Z=31), Germanium (Z=32), Arsen (Z=33), Selen (Z=34), Brom (Z=35), Rubidium (Z=37), Strontium (Z=38), Yttrium (Z=39), Zirkonium (Z=40), Niob (Z=41), Molybdän (Z=42), Technetium (Z=43), Ruthenium (Z=44), Rhodium (Z=45), Palladium (Z=46), Silber (Z=47), Cadmium (Z=48), Indium (Z=49), Zinn (Z=50), Antimon (Z=51), Tellur (Z=52), Jod (Z=53), Cäsium (Z=55), Barium (Z=56), Lanthan (Z=57), Cer (Z=58), Praseodym (Z=59), Neodym (Z=60), Promethium (Z=61), Samarium (Z=62), Europium (Z=63), Gadolinium (Z=64), Terbium (Z=65), Dysprosium (Z=66), Holmium (Z=67), Erbium (Z=68), Thulium (Z=69), Ytterbium (Z=70), Lutetium (Z=71), Hafnium (Z=72), Tantal (Z=73), Wolfram (Z=74), Rhenium (Z=75), Osmium (Z=76), Iridium (Z=77), Platin (Z=78), Gold (Z=79), Thallium (Z=81), Blei (Z=82), Wismut (Z=83), Uran (Z=92).

32. Das Strahlentherapiebehandlungssystem (100, 200) nach Anspruch 31, wobei die Nanopartikel mit hohem Z-Wert (108) aus Thulium (Z=69) und Erbium (Z=68) ausgewählt sind.

33. Das Strahlentherapiebehandlungssystem (100, 200) gemäß einem der Ansprüche 22, 31 und 32, wobei die Nanopartikel (108) mit hohem Z-Wert mindestens ein Nichtmetallelement umfassen.

34. Das Strahlentherapiebehandlungssystem (100, 200) nach Anspruch 33, wobei das mindestens eine Nichtmetallelement aus Silikon, Halogenen, Sauerstoff und Wasserstoff ausgewählt ist.

35. Das Strahlentherapiebehandlungssystem (100, 200) nach einem der Ansprüche 22, 31-32, wobei die mindestens einen Nanopartikel mit hohem Z-Wert (108) Hafniumoxid HfO₂ umfassen.

36. Das Strahlentherapiebehandlungssystem (100, 200) nach einem der Ansprüche 22 und 31-35, umfassend mindestens zwei Typen, einen ersten Typ und einen zweiten Typ, der genannten Nanopartikel (108) mit hohem Z, wobei die Nanopartikel (108) mit hohem Z des ersten Typs an Moleküle gebunden sind, die eine Affinität zu einer Art (z. B. gesunden Zellen) aufweisen, wobei die Nanopartikel (108) mit hohem Z des zweiten Typs an Moleküle gebunden sind, die eine Affinität zu einer anderen Art (z. B. nicht gesunden) von Zellen aufweisen, so dass die von den Nanopartikeln mit hohem Z-Wert des ersten Typs erzeugte XRF-Strahlung von der von den Nanopartikeln mit hohem Z-Wert (108) des zweiten Typs erzeugten XRF-Strahlung unterscheidbar ist.

37. Das Strahlentherapiebehandlungssystem (100, 200) nach einem der Ansprüche 21, 24-28, wobei der mindestens eine Röntgendetektor (202) dazu ausgelegt ist, die Strahlentherapiebehandlung in Echtzeit zu überwachen und so die Verteilung der Nanopartikel mit hohem Z-Wert (108) im Zielorgan (110) während der gesamten Behandlung kontinuierlich bereitzustellen.

## Revendications

1. Un système de traitement de radiothérapie (100) destiné à réaliser une imagerie radiographique à rayons X sur un organe cible pendant un traitement radiographique de l'organe cible, comprenant :
(i) une source de faisceau de rayons X (102) pouvant être configurée pour délivrer un faisceau de rayons X (112) à un organe cible (110),
(ii) des moyens optiques pour faire converger et mettre en forme ledit faisceau pour obtenir un faisceau de photons de rayons X en forme de cône (112) qui frappent simultanément l'organe cible (110),
(iii) de multiples nanoparticules à Z élevé (108) pouvant être administrées à l'organe cible, ou au moins un repère de centrage à Z élevé pouvant être attaché audit organe cible (110), lesdites nanoparticules à Z élevé (108) ou au moins un repère de centrage à Z élevé absorbant ledit rayonnement de rayons X et émettant des photons de fluorescence X (XRF),
(iv) au moins un détecteur XRF (106) pour détecter lesdits photons XRF éjectés hors du corps d'un patient, et
(v) des moyens de commande pour commander au moins un des composants (i) à (iv) pour commander ladite procédure de traitement de radiothérapie,
dans lequel ledit faisceau de rayons X (112) peut être focalisé sur une section dans ledit organe cible (110) ayant la concentration desdites nanoparticules à Z élevé (108) conduisant à une émission souhaitable desdits photons XRF, et
dans lequel, en cas de diminution de l'émission desdits photons XRF, ledit faisceau de rayons X (112) est mobile pour se refocaliser sur ladite section dans ledit organe cible (110) où l'émission desdits photons XRF est souhaitable,
**caractérisé en ce que** les moyens optiques comprennent au moins une lentille convergente (104, 204) pour faire converger ledit faisceau de rayons X (112) vers ledit organe cible (110).

2. Le système de traitement de radiothérapie (100) selon la revendication 1, dans lequel ledit au moins un détecteur XRF (106) est mobile.

3. Le système de traitement de radiothérapie (100) selon la revendication 1, comprenant en outre au moins une lentille convergente (104) pour faire converger lesdits photons XRF éjectés hors du corps du patient vers ledit au moins un détecteur XRF (106).

4. Le système de traitement de radiothérapie (100) selon l'une quelconque des revendications 1 à 3, dans lequel ledit au moins un détecteur XRF (106) est choisi parmi un détecteur ponctuel, un détecteur à matrice unidimensionnelle et un détecteur à matrice bidimensionnelle.

5. Le système de traitement de radiothérapie (100) selon la revendication 4, dans lequel ledit détecteur ponctuel est choisi parmi des détecteurs de type chambre d'ionisation, des détecteurs à scintillation et des détecteurs à semi-conducteurs.

6. Le système de traitement de radiothérapie (100) selon la revendication 5, dans lequel ledit détecteur à matrice bidimensionnelle est une caméra gamma.

7. Le système de traitement de radiothérapie (100) selon la revendication 1, dans lequel lesdites nanoparticules à Z élevé (108) sont choisies parmi des éléments métalliques ayant un numéro atomique d'au moins 22.

8. Le système de traitement de radiothérapie (100) selon la revendication 7, dans lequel lesdites nanoparticules à Z élevé sont choisies parmi le titane (Z = 22), le vanadium (Z = 23), le chrome (Z = 24), le manganèse (Z = 25), le fer (Z = 26), le cobalt (Z = 27), le nickel (Z = 28), le cuivre (Z = 29), le zinc (Z = 30), le gallium (Z = 31), le germanium (Z = 32), l'arsenic (Z = 33), le sélénium (Z = 34), le brome (Z = 35), le rubidium (Z = 37), le strontium (Z = 38), l'yttrium (Z = 39), le zirconium (Z = 40), le niobium (Z = 41), le molybdène (Z = 42), le technétium (Z = 43), le ruthénium (Z = 44), le rhodium (Z = 45), le palladium (Z = 46), l'argent (Z = 47), le cadmium (Z = 48), l'indium (Z = 49), l'étain (Z = 50), l'antimoine (Z = 51), le tellure (Z = 52), l'iode (Z = 53), le césium (Z = 55), le baryum (Z = 56), le lanthane (Z = 57), le cérium (Z = 58), le praséodyme (Z = 59), le néodyme (Z = 60), le prométhium (Z = 61), le samarium (Z = 62), l'europium (Z = 63), le gadolinium (Z = 64), le terbium (Z = 65), le dysprosium (Z = 66), l'holmium (Z = 67), l'erbium (Z = 68), le thulium (Z = 69), l'ytterbium (Z = 70), le lutétium (Z = 71), le hafnium (Z = 72), le tantale (Z = 73), le tungstène (Z = 74), le rhénium (Z = 75), l'osmium (Z = 76), l'iridium (Z = 77), le platine (Z = 78), l'or (Z = 79), le thallium (Z = 81), le plomb (Z = 82), le bismuth (Z = 83), l'uranium (Z = 92).

9. Le système de traitement de radiothérapie (100) selon la revendication 8, dans lequel lesdites nanoparticules à Z élevé (108) sont choisies parmi le thulium (Z = 69) et l'erbium (Z = 68).

10. Le système de traitement de radiothérapie (100) selon l'une quelconque des revendications 1 et 7 à 9, dans lequel lesdites nanoparticules à Z élevé (108) comprennent au moins un élément non métallique.

11. Le système de traitement de radiothérapie (100) selon la revendication 10, dans lequel ledit au moins un élément non métallique est choisi parmi une silicone, le carbone, les halogènes, l'oxygène et l'hydrogène.

12. Le système de traitement de radiothérapie (100) selon l'une quelconque des revendications 1 et 7 à 11, dans lequel lesdites nanoparticules à Z élevé (108) ont une forme de réseaux métallo-organiques à l'échelle nanométrique, nMOF.

13. Le système de traitement de radiothérapie (100) selon l'une quelconque des revendications 1 et 7 à 9, dans lequel lesdites au moins une nanoparticule à Z élevé (108) comprend de l'oxyde d'hafnium HfO₂.

14. Le système de traitement de radiothérapie (100) selon l'une quelconque des revendications 1 et 7 à 13, comprenant au moins deux différentes nanoparticules à Z élevé (108) : des nanoparticules à Z élevé A et des nanoparticules à Z élevé B, lesdites nanoparticules à Z élevé A pouvant être attachées à des molécules ayant une affinité vis-à-vis de cellules d'un premier type, lesdites nanoparticules à Z élevé B pouvant être attachées à des molécules ayant une affinité vis-à-vis de cellules d'un second type, dans lequel le rayonnement XRF pouvant être produit par lesdites nanoparticules à Z élevé A peut être distingué du rayonnement XRF pouvant être produit par lesdites nanoparticules à Z élevé B.

15. Le système de traitement de radiothérapie (100) selon la revendication 14, dans lequel, en cas de diminution du rayonnement XRF pouvant être produit par lesdites nanoparticules à Z élevé B et/ou d'augmentation du rayonnement XRF pouvant être produit par lesdites nanoparticules à Z élevé A pendant ladite procédure de traitement radiographique, ledit faisceau de rayons X (112) doit être refocalisé.

16. Le système de traitement de radiothérapie (100) selon l'une quelconque des revendications 14 à 15, lesdites cellules d'un premier type étant des cellules saines et lesdites cellules d'un second type étant des cellules non saines.

17. Le système de traitement de radiothérapie (100) selon l'une quelconque des revendications 1 et 2 à 6, dans lequel ledit au moins un détecteur de rayons X (106) est adapté pour surveiller en temps réel ledit traitement de radiothérapie, en assurant ainsi la répartition desdites nanoparticules à Z élevé (108) dans ledit organe cible (110) de manière continue tout au long du traitement de radiothérapie.

18. Le système de traitement de radiothérapie (100) selon l'une quelconque des revendications 1 à 17, comprenant en outre un système de simulation (300) pour simuler ladite procédure de traitement radiographique afin de maximiser la précision du traitement, ledit système de simulation fonctionnant indépendamment du système de traitement de radiothérapie.

19. Le système de traitement de radiothérapie (100) selon la revendication 18, dans lequel ledit système de simulation (300) comprend une source de rayons X (302), au moins un détecteur de rayons X (304), et de multiples nanoparticules à Z élevé (108) pouvant être attachées audit organe cible (110).

20. Le système de traitement de radiothérapie (100) selon l'une quelconque des revendications 17 à 19, dans lequel ledit système de radiothérapie est adapté pour produire des images de diagnostic en 3D dudit organe cible (110), en permettant ainsi des traitements précis.

21. Le système de traitement de radiothérapie (100) selon l'une quelconque des revendications 18 à 20, dans lequel ledit système de simulation (300) et ledit système de traitement de radiothérapie (100) peuvent être utilisés de manière interchangeable pendant un traitement pour maximiser la précision du traitement.

22. Le système de traitement de radiothérapie (100, 200) selon la revendication 1, ledit système de traitement de radiothérapie comprenant en outre :
(e) un détecteur de rayons X pour détecter ledit faisceau de rayons X traversant ledit organe cible pour simuler ledit traitement radiographique,
dans lequel ledit système de radiothérapie (200) peut basculer entre un mode de simulation et un mode de traitement de radiothérapie sans faire bouger un patient d'une position à une autre.

23. Le système de traitement de radiothérapie (100, 200) selon la revendication 22, dans lequel ladite au moins une lentille (204) comprend une ouverture ouvrable (206), ladite ouverture ouvrable (206) étant maintenue fermée pour faire converger ledit faisceau de rayons X (112) vers ledit organe cible (110) pendant ladite procédure de traitement de radiothérapie, ladite ouverture (206) étant maintenue ouverte pour permettre audit faisceau de rayons X (112) de passer à travers ladite ouverture (206) pour simuler ledit traitement radiographique.

24. Le système de traitement de radiothérapie (100, 200) selon la revendication 22, dans lequel ledit système de radiothérapie est adapté pour produire des images de diagnostic en 3D dudit organe cible (110), en permettant ainsi des traitements précis.

25. Le système de traitement de radiothérapie (100, 200) selon la revendication 22, dans lequel ledit au moins un détecteur XRF (202) est mobile.

26. Le système de traitement de radiothérapie (100, 200) selon la revendication 22, comprenant en outre au moins une lentille convergente (204) pour faire converger lesdits photons XRF éjectés hors du corps du patient vers ledit au moins un détecteur XRF (202).

27. Le système de traitement de radiothérapie (100, 200) selon l'une quelconque des revendications 22 à 25, dans lequel ledit au moins un détecteur XRF (202) est choisi parmi un détecteur ponctuel, un détecteur à matrice unidimensionnelle et un détecteur à matrice bidimensionnelle.

28. Le système de traitement de radiothérapie (100, 200) selon la revendication 26, dans lequel ledit détecteur ponctuel est choisi parmi des détecteurs de type chambre d'ionisation, des détecteurs à scintillation et des détecteurs à semi-conducteurs.

29. Le système de traitement de radiothérapie (100, 200) selon la revendication 26, dans lequel ledit détecteur à matrice bidimensionnelle est une caméra gamma.

30. Le système de traitement de radiothérapie (100, 200) selon la revendication 22, dans lequel lesdites nanoparticules à Z élevé (108) sont choisies parmi des éléments métalliques ayant un numéro atomique d'au moins 22.

31. Le système de traitement de radiothérapie (100, 200) selon la revendication 30, dans lequel lesdites nanoparticules à Z élevé (108) sont choisies parmi le titane (Z = 22), le vanadium (Z = 23), le chrome (Z = 24), le manganèse (Z = 25), le fer (Z = 26), le cobalt (Z = 27), le nickel (Z = 28), le cuivre (Z = 29), le zinc (Z = 30), le gallium (Z = 31), le germanium (Z = 32), l'arsenic (Z = 33), le sélénium (Z = 34), le brome (Z = 35), le rubidium (Z = 37), le strontium (Z = 38), l'yttrium (Z = 39), le zirconium (Z = 40), le niobium (Z = 41), le molybdène (Z = 42), le technétium (Z = 43), le ruthénium (Z = 44), le rhodium (Z = 45), le palladium (Z = 46), l'argent (Z = 47), le cadmium (Z = 48), l'indium (Z = 49), l'étain (Z = 50), l'antimoine (Z = 51), le tellure (Z = 52), l'iode (Z = 53), le césium (Z = 55), le baryum (Z = 56), le lanthane (Z = 57), le cérium (Z = 58), le praséodyme (Z = 59), le néodyme (Z = 60), le prométhium (Z = 61), le samarium (Z = 62), l'europium (Z = 63), le gadolinium (Z = 64), le terbium (Z = 65), le dysprosium (Z = 66) le holmium (Z = 67), l'erbium (Z = 68), le thulium (Z = 69), l'ytterbium (Z = 70), le lutétium (Z = 71), le hafnium (Z = 72), le tantale (Z = 73), le tungstène (Z = 74), le rhénium (Z = 75), l'osmium (Z = 76), l'iridium (Z = 77), le platine (Z = 78), l'or (Z = 79), le thallium (Z = 81), le plomb (Z = 82), le bismuth (Z = 83), l'uranium (Z = 92).

32. Le système de traitement de radiothérapie (100, 200) selon la revendication 31, dans lequel lesdites nanoparticules à Z élevé (108) sont choisies parmi le thulium (Z = 69) et l'erbium (Z = 68).

33. Le système de traitement de radiothérapie (100, 200) selon l'une quelconque des revendications 22, 31 et 32, dans lequel lesdites nanoparticules à Z élevé (108) comprennent au moins un élément non métallique.

34. Le système de traitement de radiothérapie (100, 200) selon la revendication 33, dans lequel ledit au moins un élément non métallique est choisi parmi une silicone, les halogènes, l'oxygène et l'hydrogène.

35. Le système de traitement de radiothérapie (100, 200) selon l'une quelconque des revendications 22, 31 et 32, dans lequel lesdites au moins une nanoparticule à Z élevé (108) comprend de l'oxyde d'hafnium HfO₂.

36. Le système de traitement de radiothérapie (100, 200) selon l'une quelconque des revendications 22 et 31 à 35, comprenant au moins deux types, un premier type et un second type, desdites nanoparticules à Z élevé (108), lesdites nanoparticules à Z élevé (108) dudit premier type étant attachées à des molécules ayant une affinité vis-à-vis d'une sorte (par exemple des cellules saines), lesdites nanoparticules à Z élevé (108) dudit second type étant attachées à des molécules ayant une affinité vis-à-vis d'une autre sorte de cellules (par exemple non saines), de sorte que le rayonnement XRF produit par lesdites nanoparticules à Z élevé dudit premier type peut être distingué du rayonnement XRF produit par lesdites nanoparticules à Z élevé (108) dudit second type.

37. Le système de traitement de radiothérapie (100, 200) selon l'une quelconque des revendications 21 et 24 à 28, dans lequel ledit au moins un détecteur de rayons X (202) est adapté pour surveiller en temps réel ladite procédure de traitement de radiothérapie, en assurant ainsi la répartition desdites nanoparticules à Z élevé (108) dans ledit organe cible (110) en continu tout au long du traitement de radiothérapie.
